# EUROPEAN PATENT APPLICATION

(11) **EP 4 137 485 A1**
(43) Date of publication of application: **22.02.2023**
(21) Application number: 21192395.8
(22) Date of filing: 20.08.2021
(51) Int. Cl.: C07D 307/12, C07C 41/00, C07D 307/40, C07D 407/04, C08F 16/32

(54) **VINYL ETHERS**

(71) Applicant: Henkel AG & Co. KGaA, 40589 Düsseldorf (DE)
(72) Inventor: Brandt, Adrian, 45219 Essen (DE); Beck, Horst, 41470 Neuss (DE); Taden, Andreas, 40597 Düsseldorf (DE); Spiegelberg, Brian, 40627 Düsseldorf (DE); de Vries, Johannes Gerardus, 18055 Rostock (DE)

(57) **Abstract**

The present invention relates to a method for producing at least one vinyl ether compound having at least one cyclic structure using at least one Iridium catalyst, at least one base and at least one solvent selected from ester and/or ether solvents. Furthermore, the present invention relates to a respective vinyl ether, to a polymer obtainable by polymerizing this vinyl ether compound according to the invention, to an adhesive comprising the at least one polymer according to the invention and to the use of at least one vinyl ether compound according to the invention or at least one polymer according to the invention for the production of UV adhesives, cationic curing or 1-component or 2-component systems.

## Description

The present invention relates to a method for producing at least one vinyl ether compound having at least one cyclic structure using at least one Iridium catalyst, at least one base and at least one solvent selected from ester and/or ether solvents. Furthermore, the present invention relates to a respective vinyl ether, to a polymer obtainable by polymerizing this vinyl ether compound according to the invention, to an adhesive comprising the at least one polymer according to the invention and to the use of at least one vinyl ether compound according to the invention or at least one polymer according to the invention for the production of UV adhesives, cationic curing or 1-component or 2-component systems.

UV adhesives and reactive adhesives are widely used in the art and are versatile adhesives used for many different applications, including but not limited to automotive industry, furniture, veneers, electronics and the like. Most of the existing processes for producing compounds suitable for this type of adhesive are not based on sustainable and environmentally friendly techniques.

Thus, there is need in the art, for alternative manufacturing processes that can overcome the known drawbacks and provide compounds in economically sufficient yield, based on renewable resources for sustainable adhesive systems with a good performance.

Therefore, the present invention is based on the inventors' finding that mono- and divinyl ether compounds are obtainable by a specific method starting from, preferably at least one bio-based substrate, in the presence of a catalyst, a base and a solvent selected from ester and/or ether solvents.

The present invention relates to a method for producing at least one vinyl ether compound having at least one cyclic structure, wherein the method comprises or consists of the steps:
reacting a vinyl ester with an alcohol having at least one cyclic structure and at least one hydroxy group, preferably one or two hydroxy groups, in the presence of at least one Iridium catalyst, at least one base and at least one solvent selected from ester and/or ether solvents.

With this method a highly efficient synthesis towards vinyl ethers with a cyclic structure from any kind of cyclic alcohol is presented. Especially the vinylation of hydroxy groups directly or by a CH₂ group linked to a cyclic structure is highly efficient, avoiding almost any acetal formation. In case of diols, this method effectively yields divinyl ethers with a cyclic structure.

In a preferred embodiment, the present invention relates to a method for producing at least one vinyl ether of formula (I) or (II) 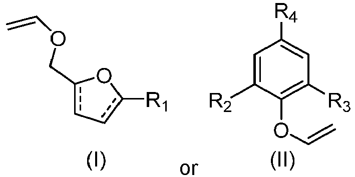 wherein
R₁ is selected from a substituted or unsubstituted C₁-C₁₀ alkyl or C₃-C₁₀ alkenyl or C₂-C₁₀ alkoxy group; "-----" can be a single or double bond; and
R₂, R₃ and R₄ are independently selected from -H, a substituted or unsubstituted C₁-C₁₀ alkyl or C₂-C₁₀ alkenyl or C₂-C₁₀ alkoxy group, with the provision that only one of R₂, R₃ and R₄ is a hydrogen;
wherein the method comprises or consists of the following steps:
reacting a compound of formula (III) 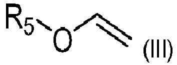 wherein R₅ is -C(=O)R₁₀, and R₁₀ is an unsubstituted C₁-C₁₀ alkyl group; preferably the compound of formula (III) is vinyl acetate,
with a compound of formula (IV) or formula (V) 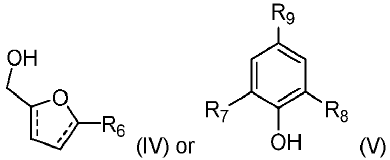 wherein
R₆ is selected from a substituted or unsubstituted C₁-C₁₀ alkyl or C₃-C₁₀ alkenyl or C₂-C₁₀ alkoxy group; "-----" can be a single or double bond; and
R₇, R₈ and R₉ are independently selected from -H, a substituted or unsubstituted C₁-C₁₀ alkyl or C₂-C₁₀ alkenyl or C₂-C₁₀ alkoxy group, with the provision that only one of R₇, R₈ and R₉ is a hydrogen;
in the presence of at least one Iridium catalyst, at least one base and at least one solvent selected from ester and/or ether solvents.

In various embodiments, in the compound of formula (I) and (II)
R₁ is selected from a substituted or unsubstituted C₁-C₁₀ alkyl or C₃-C₁₀ alkenyl, -CH₂-OH or -CH₂-O-CH=CH₂; -CH-(O-CH₂-)₂-CH-O-CH=CH₂; preferably R₁ is selected from -CH₃, -CH₂-OH or -CH₂-O-CH=CH₂ and/or
both "-----" are a single or a double bond,
R₂ is -H, -CH₃, or -O-CH₃ and/or
R₃ is -H, -CH₃, or -O-CH₃ and/or
R₄ is -H, -C₂H₃ or -C₃H₅;
wherein only one of R₂, R₃ and R₄ is a hydrogen; and/or
wherein in the compound of formula (IV) and (V)
R₆ is selected from a substituted or unsubstituted C₁-C₁₀ alkyl or C₃-C₁₀ alkenyl, -CH₂-OH; -CH-(O-CH₂-)₂-CH-OH; preferably R₁ is selected from -CH₃ or -CH₂-OH and/or
both "-----" are a single or a double bond,
R₇ is -H, -CH₃, or -O-CH₃ and/or
R₈ is -H, -CH₃, or -O-CH₃ and/or
R₉ is -H, -C₂H₃ or -C₃H₅;
wherein only one of R₇, R₈ and R₉ is a hydrogen;

In various embodiments, the reaction temperature of the method according to the invention is between 50 to 150 °C, preferably between 60 to 90°C or 90 to 120 °C.

In various embodiments, the reaction time of the method according to the invention is between 1 to 15 h, preferably between 4 to 10 h.

In various embodiments of the method according to the invention,
(i) the at least one Iridium catalyst is selected from Chlorobis(cyclooctene)iridium(I) dimer, Chloro-1,5-cyclooctadiene iridium(I) dimer,1,5-Cyclooctadiene(acetylacetonato)iridium(I), 1,5-Cyclooctadiene(hexafluoroacetylacetonato)iridium(I), 1,5-Cyclooctadiene(η5-indenyl)iridium(I), Di-µ-methoxobis(1,5-cyclooctadiene)diiridium(I), (Methylcyclopentadienyl)(1,5-cyclooctadiene)iridium(I), Chloro(1,5-cyclooctadiene)(1,10-phenanthroline)iridium(I) THF adduct, Bis(pyridine)(1,5-cyclooctadiene)iridium(I) hexafluorophosphate, Bis(1,5-cyclooctadiene)iridium(I) tetrakis[3,5-bis(trifluoromethyl)phenyl]borate, Bis(1,5-cyclooctadiene)iridium(I) tetrafluoroborate, Bis(acetonitrile)(1,5-cyclooctadiene)iridium(I) tetrafluoroborate, Bis(1,5-cyclooctadiene)iridium(I) hexafluorophosphate, (Tricyclohexylphosphine)(1,5-cyclooctadiene)(pyridine)iridium(I)hexafluorophosphate, more preferably cationic Ir(I) complexes like Chlorobis(cyclooctene)iridium(I) dimer, Bis(pyridine)(1,5-cyclooctadiene)iridium(I) hexafluorophosphate, Bis(1,5-cyclooctadiene)iridium(I) tetrakis[3,5-bis(trifluoromethyl)phenyl]borate, Bis(1,5-cyclooctadiene)iridium(I) tetrafluoroborate, Bis(acetonitrile)(1,5-cyclooctadiene)iridium(I) tetrafluoroborate, Bis(1,5-cyclooctadiene)iridium(I) hexafluorophosphate, (Tricyclohexylphosphine)(1,5-cyclooctadiene)(pyridine)iridium(I)hexafluorophosphate most preferably Chlorobis(cyclooctene)iridium(I) dimer and Bis(1,5-cyclooctadiene)iridium(I) tetrafluoroborate ; and/or
(ii) the at least one base is selected from sodium carbonate, potassium carbonate, cesium carbonate, potassium phosphate, sodium phosphate, sodium benzoate, potassium benzoate, sodium formate, potassium formate, sodium methoxide, potassium methoxide, sodium ethoxide, potassium ethoxide, sodium-tertbutoxide, potassium-tertbutoxide, sodium trimethylacetate, sodium acetate or potassium acetate, more preferably selected from sodium acetate or potassium acetate; and/or
(iii) the least one solvent is selected from lactons, like gamma-valerolacton, ether solvents, like cyclopentyl methyl ether, methyl tert-butyl ether, tetrahydrofuran, 2-methyl tetrahydrofuran, 3-methyl tetrahydrofuran, 2,5-dimethyltetrahydrofuran, 2,2,5,5-tetramethyltetrahydrofuran, most preferably the solvent is selected from tetrahydrofuran and 2-methyl tetrahydrofuran, especially the solvent is 2-methyl tetrahydrofuran.

In addition, the method according to the invention can be carried out under protective atmosphere (nitrogen, argon).

In various embodiments of the method according to the invention, the vinyl ester, especially the compound of formula (III), is added in molar excess over the alcohol, preferably in a molar equivalent ratio of at least 1:1, (>1:1), more preferably of at least 2:1, most preferably in a ratio of 3:1 (vinyl ester: alcohol).

In further embodiments of the method according to the invention, the vinyl ether being the reaction product of a vinyl ester and a alcohol having two hydroxy groups, especially compound of formula (I) is a mixture of divinyl ether and mono vinyl ether compounds, preferably the mixture comprises at least 50 wt.-% divinyl ether, more preferably at least 60 wt.-%, more preferably at least 70 wt.-%, most preferably at least 80 wt.-%, based on the total weight of the mixture.

In preferred embodiments, the alcohol, especially the compound of formula (IV) or formula (V) is bio-based.

In a second aspect, the present invention relates to a vinyl ether compound of formula (I) or (II) 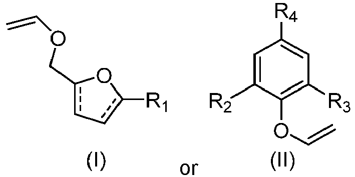 wherein
R₁ is selected from a substituted or unsubstituted C₁-C₁₀ alkyl or C₃-C₁₀ alkenyl or C₂-C₁₀ alkoxy group; "-----" can be a single or double bond; preferably R₁ is selected from a substituted or unsubstituted C₁-C₁₀ alkyl or C₃-C₁₀ alkenyl, -CH₂-OH or -CH₂-O-CH=CH₂; -CH-(O-CH₂-)₂-CH-O-CH=CH₂; preferably R₁ is selected from -CH₃, -CH₂-OH or -CH₂-O-CH=CH₂ and/or both "-----" are a single or a double bond; and
R₂, R₃ and R₄ are independently selected from -H, a substituted or unsubstituted C₁-C₁₀ alkyl or C₂-C₁₀ alkenyl or C₂-C₁₀ alkoxy group, with the provision that only one of R₂, R₃ and R₄ is a hydrogen; preferably R₂ is -H, -CH₃, or -O-CH₃, R₃ is -H, -CH₃, or -O-CH₃ and R₄ is -H, -C₂H₃ or -C₃H₅, wherein only one of R₂, R₃ and R₄ is a hydrogen; or
the vinyl ether compound is any one of the compounds of formula (VI) to (XIV)

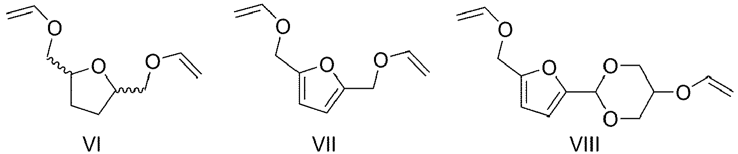

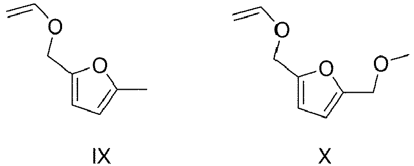

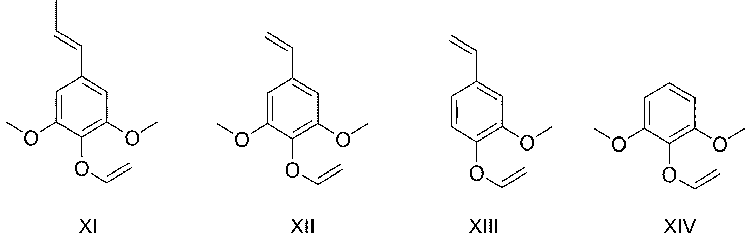

Especially preferred are the vinyl ether compounds of formulae (VI), (VII) and (XIII).

In a third aspect, the present invention relates to a polymer obtainable by polymerizing a vinyl ether compound of formula (I) or (II) according to the invention optionally together with at least one comonomer.

In a fourth aspect, the present invention relates to an adhesive comprising at least one polymer according to the invention.

Finally, in a fifth aspect, the present invention relates to a use of at least one vinyl ether compound of formula (I) or (II) according to the invention or at least one polymer according to the invention for the production of UV adhesives, cationic curings, or 1-component or 2-component systems, e.g. can coatings, epoxy- and acrylate-based coatings, structural adhesives, sealants, battery adhesives, wood coatings or pressure sensitive adhesives (PSA)).

The details of one or more embodiments are set forth in the accompanying drawings and the description below. Other features, objects, and advantages will be apparent from the description, drawings and examples, and from the claims.

The following detailed description refers to, by way of illustration, specific details and embodiments in which the invention may be practiced. These embodiments are described in sufficient detail to enable those skilled in the art to practice the invention. Other embodiments may be utilized and structural and logical changes may be made without departing from the scope of the invention. The various embodiments are not necessarily mutually exclusive, as some embodiments can be combined with one or more other embodiments to form new embodiments.

Unless otherwise defined, all technical and scientific terms used herein have the same meaning as commonly understood by one of ordinary skill in the art. The singular terms "a", "an" and "the" include plural referents unless context clearly indicates otherwise.

"At least one", as used herein, means one or more, i.e. 1, 2, 3, 4, 5, 6, 7, 8, 9 or more of the referenced species. Similarly, "one or more", as used herein, relates to at least one and comprises 1, 2, 3, 4, 5, 6, 7, 8, 9 or more. In connection with a given species, the term does not relate to the total number of molecules, but rather to the type of species, e.g. vinyl ether compounds. In connected with amounts, the term relates to the total amount of the referenced species.

Numeric values specified without decimal places refer to the full value specified with one decimal place, i.e. for example, 99 % means 99.0 %, unless otherwise defined.

The terms "about" or "approximately" or "approx.", in connection with a numerical value, refer to a variance of ±10 %, preferably ±5 %, more preferably ±2 %, more preferably ±1 %, and most preferably less than ±1 %, with respect to the given numerical value.

When an amount, a concentration or other values or parameters is/are expressed in form of a range, a preferable range, or a preferable upper limit value and a preferable lower limit value, it should be understood as that any ranges obtained by combining any upper limit or preferable value with any lower limit or preferable value are specifically disclosed, without considering whether the obtained ranges are clearly mentioned in the context.

According to the first aspect, the present invention relates to a method for producing at least one vinyl ether compound having at least one cyclic structure, wherein the method comprises or consists of the steps:
reacting a vinyl ester with an alcohol having at least one cyclic structure and at least one hydroxy group, preferably one or two hydroxy groups, in the presence of at least one Iridium catalyst, at least one base and at least one solvent selected from ester and/or ether solvents.

The alcohol having at least one cyclic structure and at least one hydroxy group can be any alcohol which contains at least one cyclic structure. The cyclic structure can be cycloaliphatic or aromatic, like a cyclopentane group, cyclohexane group, an adamantane group, a norbornene group, a cyclopentadiene group or a phenyl group. The cyclic structure can also contain heteroatoms, especially oxygen, like in a furan group or a tetrahydrofuran group.

In a preferred embodiment, the alcohol has at least one heterocyclic structure, especially at least one furan group and/or tetrahydrofuran group, and/or has at least one hydroxy group, especially at least two hydroxy groups, preferably exactly two hydroxy groups. In case of a tetrahydrofuran the substituents can be cis, trans or mixtures thereof. For example, in case of 2,5-bis(hydroxymethyl)tetrahydrofuran the cis-isomer, the trans-isomer and mixtures thereof can be effectively vinylated.

In another preferred embodiment, the alcohol has at least one aromatic structure, especially at least one phenyl group, preferably one phenyl group, and/or has at least one hydroxy group, preferably one hydroxy group.

In a preferred embodiment, the present invention relates to a method for producing at least one vinyl ether compound of formula (I) or (II) 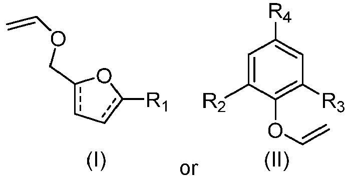 wherein
R₁ is selected from a substituted or unsubstituted C₁-C₁₀ alkyl or C₃-C₁₀ alkenyl or C₂-C₁₀ alkoxy group;
"-----" can be a single or double bond; and
R₂, R₃ and R₄ are independently selected from -H, a substituted or unsubstituted C₁-C₁₀ alkyl or C₂-C₁₀ alkenyl or C₂-C₁₀ alkoxy group, with the provision that only one of R₂, R₃ and R₄ is a hydrogen;
wherein the method comprises or consists of the following steps:
reacting a compound of formula (III) 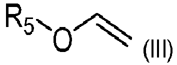 wherein R₅ is -C(=O)R₁₀, and R₁₀ is an unsubstituted C₁-C₁₀ alkyl group; preferably the compound of formula (III) is vinyl acetate,
with a compound of formula (IV) or formula (V) 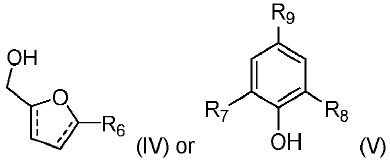 wherein
R₆ is selected from a substituted or unsubstituted C₁-C₁₀ alkyl or C₃-C₁₀ alkenyl or C₂-C₁₀ alkoxy group; "-----" can be a single or double bond; and
R₇, R₈ and R₉ are independently selected from -H, a substituted or unsubstituted C₁-C₁₀ alkyl or C₂-C₁₀ alkenyl or C₂-C₁₀ alkoxy group, with the provision that only one of R₇, R₈ and R₉ is a hydrogen;
in the presence of at least one Iridium catalyst, at least one base and at least one solvent selected from ester and/or ether solvents.

In various embodiments of the method according to the invention, the compound of formula (III) is bio-based.

In general, unless otherwise stated, bio-based materials or biomaterials include or are preferably made of materials, chemicals and energy derived from renewable biological resources, for example from living organisms such as plants, bacteria or animals, without being limited to these. "Bio-based", as used in this context, thus refers to compounds/materials that can be obtained from biological, i.e. renewable, sources. Examples for a cyclic alcohol compound of formula (IV) are bio-based bis(hydroxymethyl)furan or bis(hydroxymethyl)tetrahydrofuran.

In one embodiment of the method according to the invention, vinyl ether(s) according to the invention are prepared from bio-based materials, preferably from bio-based diols, most preferably from bio-based bis(hydroxymethyl)furan or bis(hydroxymethyl)tetrahydrofuran.

Typically, the term "C₁-C₁₀ alkyl" comprises linear and branched alkyl groups, wherein linear alkyl groups comprise 1 to 10, preferably 1 to 6 C-atoms and branched alkyl groups comprise 3 to 10, preferably 3 to 6 C-atoms. In addition, the term "C₂-C₁₀ alkenyl" comprises linear and branched alkenyl groups, wherein linear alkenyl groups comprise 2 to 10, preferably 2 to 6 C-atoms and branched alkenyl groups comprise 4 to 10, preferably 4 to 6 C-atoms.

In general, in substituted alkyl or alkenyl groups, each H can be independently substituted with a halogen group (e.g. -F, -CI, -Br or- I), -OH, -O-CH₃, -O-CH₂-CH₃, or -NO₂. It is however preferred that in various embodiments these groups are unsubstituted.

In various embodiments, in vinyl ether compounds of formula (I) and (II)
R₁ is selected from a substituted or unsubstituted C₁-C₁₀ alkyl or C₃-C₁₀ alkenyl, -CH₂-OH or -CH₂-O-CH=CH₂; -CH-(O-CH₂-)₂-CH-O-CH=CH₂; preferably R₁ is selected from -CH₃, -CH₂-OH or -CH₂-O-CH=CH₂ and/or
both "-----" are a single or a double bond,
R₂ is -H, -CH₃, or -O-CH₃ and/or
R₃ is -H, -CH₃, or -O-CH₃ and/or
R₄ is -H, -C₂H₃ or -C₃H₅;
wherein only one of R₂, R₃ and R₄ is a hydrogen; and/or
wherein in the compound of formula (IV) and (V)
R₆ is selected from a substituted or unsubstituted C₁-C₁₀ alkyl or C₃-C₁₀ alkenyl, -CH₂-OH; -CH-(O-CH₂-)₂-CH-OH; preferably R₁ is selected from -CH₃ or -CH₂-OH and/or
both "-----" are a single or a double bond,
R₇ is -H, -CH₃, or -O-CH₃ and/or
R₈ is -H, -CH₃, or -O-CH₃ and/or
R₉ is -H, -C₂H₃ or -C₃H₅;
wherein only one of R₇, R₈ and R₉ is a hydrogen.

In various embodiments of the method according to the invention, the compound of formula (III) is added in molar excess over the compound of formula (IV) or (V), preferably in a molar equivalent ratio of at least 1:1, (>1:1), more preferably of at least 2:1, most preferably in a ratio of 3:1 (formula (III):formula (IV) or (V)).

In various embodiments of the method according to the invention,
(i) the at least one Iridium catalyst is selected from Chlorobis(cyclooctene)iridium(I) dimer, Chloro-1,5-cyclooctadiene iridium(I) dimer,1,5-Cyclooctadiene(acetylacetonato)iridium(I), 1,5-Cyclooctadiene(hexafluoroacetylacetonato)iridium(I), 1,5-Cyclooctadiene(η5-indenyl)iridium(I), Di-µ-methoxobis(1,5-cyclooctadiene)diiridium(I), (Methylcyclopentadienyl)(1,5-cyclooctadiene)iridium(I), Chloro(1,5-cyclooctadiene)(1,10-phenanthroline)iridium(I) THF adduct, Bis(pyridine)(1,5-cyclooctadiene)iridium(I) hexafluorophosphate, Bis(1,5-cyclooctadiene)iridium(I) tetrakis[3,5-bis(trifluoromethyl)phenyl]borate, Bis(1,5-cyclooctadiene)iridium(I) tetrafluoroborate, Bis(acetonitrile)(1,5-cyclooctadiene)iridium(I) tetrafluoroborate, Bis(1,5-cyclooctadiene)iridium(I) hexafluorophosphate, (Tricyclohexylphosphine)(1,5-cyclooctadiene)(pyridine)iridium(I)hexafluorophosphate, more preferably cationic Ir(I) complexes like Chlorobis(cyclooctene)iridium(I) dimer, Bis(pyridine)(1,5-cyclooctadiene)iridium(I) hexafluorophosphate, Bis(1,5-cyclooctadiene)iridium(I) tetrakis[3,5-bis(trifluoromethyl)phenyl]borate, Bis(1,5-cyclooctadiene)iridium(I) tetrafluoroborate, Bis(acetonitrile)(1,5-cyclooctadiene)iridium(I) tetrafluoroborate, Bis(1,5-cyclooctadiene)iridium(I) hexafluorophosphate, (Tricyclohexylphosphine)(1,5-cyclooctadiene)(pyridine)iridium(I)hexafluorophosphate most preferably Chlorobis(cyclooctene)iridium(I) dimer and Bis(1,5-cyclooctadiene)iridium(I) tetrafluoroborate ; and/or
(ii) the at least one base is selected from sodium carbonate, potassium carbonate, cesium carbonate, potassium phosphate, sodium phosphate, sodium benzoate, potassium benzoate, sodium formate, potassium formate, sodium methoxide, potassium methoxide, sodium ethoxide, potassium ethoxide, sodium-tertbutoxide, potassium-tertbutoxide, sodium acetate or potassium acetate, more preferably selected from sodium acetate or potassium acetate; and/or
(iii) the least one solvent is selected from lactons, like gamma-valerolacton, ether solvents, like cyclopentyl methyl ether, methyl tert-butyl ether, tetrahydrofuran, 2-methyl tetrahydrofuran, 3-methyl tetrahydrofuran, 2,5-dimethyltetrahydrofuran, 2,2,5,5-tetramethyltetrahydrofuran, most preferably the solvent is selected from tetrahydrofuran and 2-methyl tetrahydrofuran, especially the solvent is 2-methyl tetrahydrofuran.

In various embodiments, the catalyst is added in amounts of 0.01 to 2 mol%, preferably in 0.1 to 1.5 mol%, more preferably in 0.5 to 1.1 mol% based on the amount of the alcohol of formula (III).

In various embodiments, the base is added in amounts of 1 to 100 mol%, preferably 5 to 70 mol%, more preferably 10 to 50 mol%, based on the amount of the alcohol of formula (III).

In various embodiments, the reaction temperature of the method according to the invention is between 50 to 150 °C, preferably between 60 to 90°C or 90 to 120 °C. In specific, non-limiting embodiments according to the invention, the reaction temperature is 80 °C or especially 100 °C.

In various embodiments, the reaction time of the method according to the invention is between 1 to 15 h, preferably between 4 to 10 h. Typically, the reaction time can be reduced by increasing the temperature during the course of the reaction.

In various embodiments of the method according to the invention, the vinyl ether compound of formula (I) is a mixture of divinyl ether and mono vinyl ether compounds, preferably the mixture comprises at least 50 wt.-% divinyl ether, more preferably at least 60 wt.-%, more preferably at least 70 wt.-%, most preferably at least 80 wt.-%, based on the total weight of the mixture.

In various embodiments of the method according to the invention, the method comprises a further step of product purification. Examples include, without limitation filtration, column chromatography, vacuum evaporation/distillation, with evaporation/distillation being preferred. These steps are commonly known to the skilled person in the art.

According to the second aspect, the present invention relates to a vinyl ether compound of formula (I) or (II) 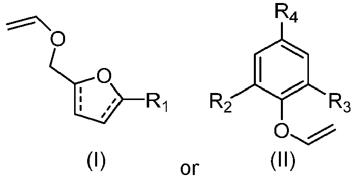 wherein
R₁ is selected from a substituted or unsubstituted C₁-C₁₀ alkyl or C₃-C₁₀ alkenyl or C₂-C₁₀ alkoxy group; "-----" can be a single or double bond; preferably R₁ is selected from a substituted or unsubstituted C₁-C₁₀ alkyl or C₃-C₁₀ alkenyl, -CH₂-OH or -CH₂-O-CH=CH₂; -CH-(O-CH₂-)₂-CH-O-CH=CH₂; preferably R₁ is selected from -CH₃, -CH₂-OH or -CH₂-O-CH=CH₂ and/or both "-----" are a single or a double bond; and
R₂, R₃ and R₄ are independently selected from -H, a substituted or unsubstituted C₁-C₁₀ alkyl or C₂-C₁₀ alkenyl or C₂-C₁₀ alkoxy group, with the provision that only one of R₂, R₃ and R₄ is a hydrogen; preferably R₂ is -H, -CH₃, or -O-CH₃, R₃ is -H, -CH₃, or -O-CH₃ and R₄ is -H, -C₂H₃ or -C₃H₅, wherein only one of R₂, R₃ and R₄ is a hydrogen; or
the vinyl ether compound is any one of the compounds of formula (VI) to (XIV)

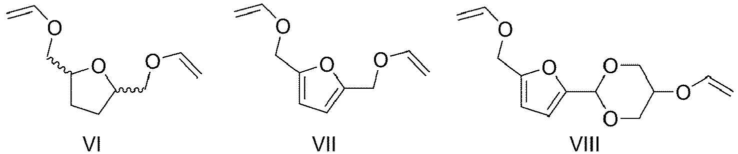

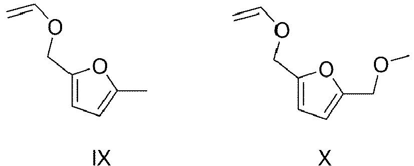

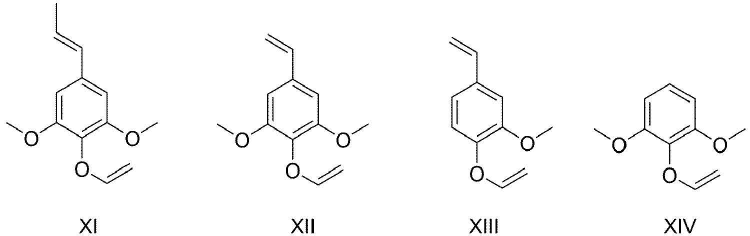

Especially preferred are the vinyl ether compounds of formulae (VI), (VII) and (XIII).

According to the third aspect, the present invention relates to a polymer obtainable by polymerizing a vinyl ether compound of formula (I) or (II) according to the invention optionally together with at least one comonomer. Generally, all commonly known comonomers, which can undergo a reaction with a vinyl group are suitable. While cationic polymerization is preferred, thiol-ene reaction and polymerization is similarly possible. Radical polymerization is relevant for vinyl ether-based dispersions or when using vinyl ether monomers as comonomers in radical polymerization with (meth)acrylates

The at least one comonomer may be a further vinyl ether different from formula (I) or (II), e.g. an alkyl vinyl ether or a hydroxy(alkyl) vinyl ether, and/or vinyl lactam and/or vinyl amid and/or isocyanate terminated polyol and/or isocyanate terminated polyol endcapped with hydroxyalkyl vinyl ether, more preferably the at least one comonomer is selected from the group of methyl vinyl ether, ethyl vinyl ether, allyl vinyl ether, propyl vinyl ether, isopropyl vinyl ether, butyl vinyl ether, isobutyl vinyl ether, *tert*-butyl vinyl ether, 2-ethylhexyl vinyl ether (EHVE), hydroxyethyl vinyl ether (HEVE), hydroxybutyl vinyl ether or ethoxylated hydroxybutyl vinyl ether with 20-125 EO-units, aminopropyl vinyl ether, phenyl vinyl ether, benzyl vinyl ether, cyclohexyl vinyl ether, butandiol divinyl ether, diethyleneglycol divinyl ether, tri(ethylene glycol) methyl vinyl ether, triethylenglycol divinyl ether, dodecyl vinyl ether, octadecyl vinyl ether, cyclohexane dimethanol divinyl ether (CHDVE), cyclohexane dimethanol mono vinyl ether (CHMVE), trimethylolpropane trivinyl ether (TMPTVE), N-vinyl formamide, N-vinyl pyrrolidone, N-vinyl caprolactame (NVC) or liquid N-vinyl caprolactame (LNVC), N,N'-divinyl-2-imidazolidone, N-vinylimidazole, vinylcarbazol, or isocyanate terminated polyols or isocyanate terminated polyols that are further endcapped with hydroxyalkyl vinyl ether derived from toluene-2,4-diisocyanate (TDI), diphenylmethane diisocyanate (MDI), hexamethylene diisocyanate (HMDI), isophorone diisocyanate (IPDI), pentamethylene diisocyanate (PDI), dicyclohexylmethane-4,4'-diisocyanate (H12MDI), *meta-*tetramethylxylylene diisocyanate (TMXDI) or polymeric diphenylmethane diisocyanate (PMDI).

In various embodiments, more than one co-monomer is present, preferably 2, 3, 4, 5, 6, 7, 8, 9, 10 or more, preferably selected from the list above, but not limited thereto.

Generally, all polymerization techniques, in which a vinyl group can undergo a polymerization reaction are suitable. For example, the mixture of monomers may be polymerized by free radical polymerization to form a vinyl ether polymer or copolymer, without being limited to these methods. Exemplary polymerization initiators include organic azo or peroxo compounds. Suitable polymerization initiators are widely known in the art and readily available. In various embodiments, the polymerization initiator comprises or consists of 2,2'-azobisisobutyronitrile (AIBN). Other suitable initiators include redox initiators, which are widely used and well-known in the art. In various preferred embodiments, the polymerization occurs by cationic polymerization or thiol-ene addition reaction and polymerization. Suitable cationic initiators are known to those skilled in the art and include, without limitation, Brønstedt acids, Lewis acids or super acid generators, such as iodonium or sulfonium salts, which can optionally be activated by UV irradiation.

The polymerization reaction can be carried out by using the vinyl ether compound(s) as defined above, and adding a cationic and/or radical initiator, as detailed above. Additional monomers that can optionally be added, such as further co-monomers, can be added during the polymerization process.

The polymerization can be carried out at elevated temperature, preferably at about 70°C, for several hours, preferably 1 to 12 hours.

After the polymerization is complete, the solvent can be removed by applying heat and/or vacuum to the mixture. Different heating temperatures and/or vacuum settings are suitable depending for example, on the used solvent. In various embodiments, the solvent is evaporated such that residual solvent content in the copolymer is between about 1 wt.-% to 35 wt.-%, preferably about 20 to 25 wt.-% relative to the reaction mixture.

After the polymerization is complete, remaining free monomers can be removed by applying heat and/or vacuum to the mixture. For this, suitable heating temperatures and/or vacuum settings can be applied depending on the type of monomer used, with such conditions being easily determinable for those skilled in the art.

The correspondingly obtained polymers can be used in adhesives, preferably UV adhesives. Such adhesive formulations can additionally contain one or more further components, which are commonly known to those skilled in the art. In specific embodiments, these additional components are selected from fillers, thickeners, silane additives, colorants, perfumes, preservatives, resins and mixtures thereof.

Therefore, according to the fourth aspect, the present invention relates to an adhesive comprising at least one polymer according to the invention.

In various embodiments, the adhesive is an UV adhesive.

Finally in a fifth aspect, the present invention relates to the use of at least one vinyl ether compound of formula (I) according to the invention or at least one polymer according to the invention for the production of UV adhesives, cationic curings, or 1-component or 2-component systems, e.g. can coatings, epoxy- and acrylate-based coatings, structural adhesives, sealants, battery adhesives, wood coatings or pressure sensitive adhesives (PSA)).

All embodiments and examples described above for the method according to the invention may also apply to other aspects according to the invention, and vice versa.

In the following, the invention is described in more detail by reference to the examples. It is understood that these examples are for illustrative purposes only and that it is not intended that the invention is limited thereto.

### EXAMPLES

### Example 1:

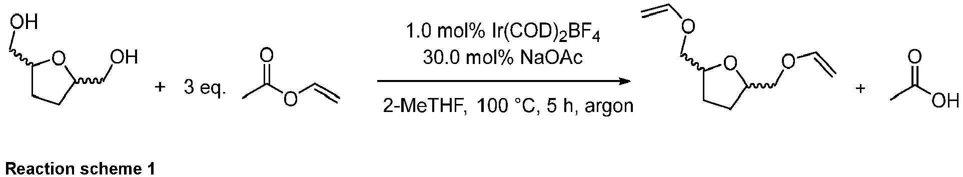

A Schlenk tube (25 mL) is charged with a magnetic stirring bar, 1.0 mol% (0.0001 mol, 50 mg) of Ir(COD)BF₄ and 30.0 mol% (0.003 mol, 246.1 mg) of NaOAc (anhydrous). Then (0.099 mol, 10 mL) freshly distilled 2-MeTHF and (0.03 mol, 2.8 mL) degassed vinyl acetate is added. Afterwards, the degassed substrate 2,5-bis(hydroxymethyl)tetrahydrofuran (0.01 mol, 1.32 g) is added *via* syringe. The Schlenk tube is sealed under argon and transferred to a preheated oil bath at 100 °C in which the reaction mixture is stirred for 5 h. Subsequently, the mixture is cooling down to room temperature and then 2-MeTHF and the excess of vinyl acetate are removed under reduced pressure on the rotary evaporator. The desired vinyl ether is purified by column chromatography using Et₂O/n-pentane (1:10/ v:v) as eluent on silica. After purification, the solvents are removed under reduced pressure on the rotary evaporator and the final compound is further dried at 40 °C at 150 mbar for 4 h. **Yield: 1.64 g, 89%**

### Example 2:

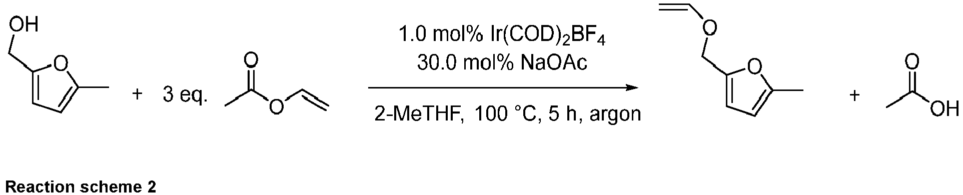

A Schlenk tube (25 mL) is charged with a magnetic stirring bar, 1.0 mol% (0.0001 mol, 50 mg) of Ir(COD)BF₄ and 30.0 mol% (0.003 mol, 246.1 mg) of NaOAc (anhydrous). Then (0.099 mol, 10 mL) freshly distilled 2-MeTHF and (0.03 mol, 2.8 mL) degassed vinyl acetate is added. Afterwards, the degassed substrate 5-methylfurfuryl alcohol (0.01 mol, 1.12 g) is added *via* syringe. The Schlenk tube is sealed under argon and transferred to a preheated oil bath at 100 °C in which the reaction mixture is stirred for 5 h. Subsequently, the mixture is cooling down to room temperature and then 2-MeTHF and the excess of vinyl acetate are removed under reduced pressure on the rotary evaporator. The desired vinyl ether is purified by column chromatography using Et₂O/n-pentane (1:10/ v:v) as eluent on silica. After purification, the solvents are removed under reduced pressure on the rotary evaporator and the final compound is further dried at 40 °C at 150 mbar for 4 h. **Yield: 1.21 g, 88%**

### Example 3:

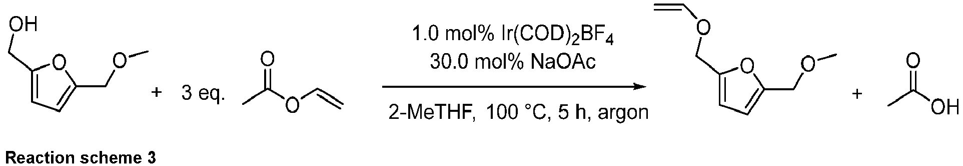

A Schlenk tube (25 mL) is charged with a magnetic stirring bar, 1.0 mol% (0.0001 mol, 50 mg) of Ir(COD)BF₄ and 30.0 mol% (0.003 mol, 246.1 mg) of NaOAc (anhydrous). Then (0.099 mol, 10 mL) freshly distilled 2-MeTHF and (0.03 mol, 2.8 mL) degassed vinyl acetate is added. Afterwards, the degassed substrate 5-(Methoxymethyl)-2-furanmethanol (0.01 mol, 1.42 g) is added *via* syringe. The Schlenk tube is sealed under argon and transferred to a preheated oil bath at 100 °C in which the reaction mixture is stirred for 5 h. Subsequently, the mixture is cooling down to room temperature and then 2-MeTHF and the excess of vinyl acetate are removed under reduced pressure on the rotary evaporator. The desired vinyl ether is purified by column chromatography using Et₂O/n-pentane (1:10/ v:v) as eluent on silica. After purification, the solvents are removed under reduced pressure on the rotary evaporator and the final compound is further dried at 40 °C at 150 mbar for 4 h. **Yield: 1.30 g, 77%**

### Example 3:

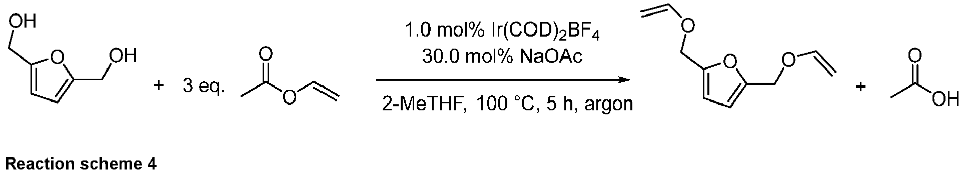

A Schlenk tube (25 mL) is charged with a magnetic stirring bar, 1.0 mol% (0.0001 mol, 50 mg) of Ir(COD)BF₄, 30.0 mol% (0.003 mol, 246.1 mg) of NaOAc (anhydrous) and 2,5-bis(hydroxymethyl)furan (0.01 mol, 1.28 g). Then (0.099 mol, 10 mL) freshly distilled 2-MeTHF and (0.03 mol, 2.8 mL) degassed vinyl acetate is added. The Schlenk tube is sealed under argon and transferred to a preheated oil bath at 100 °C in which the reaction mixture is stirred for 5 h. Subsequently, the mixture is cooling down to room temperature and then 2-MeTHF and the excess of vinyl acetate are removed under reduced pressure on the rotary evaporator. The desired vinyl ether is purified by column chromatography using Et₂O/n-pentane (1:10/ v:v) as eluent on silica. After purification, the solvents are removed under reduced pressure on the rotary evaporator and the final compound is further dried at 40 °C at 50 mbar for 4 h.

### Yield: 1.30 g, 72%

### Example 4:

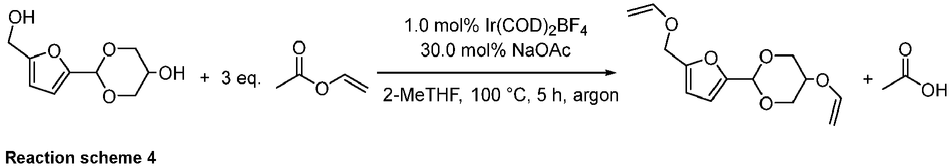

A Schlenk tube (25 mL) is charged with a magnetic stirring bar, 1.0 mol% (0.0001 mol, 50 mg) of Ir(COD)BF₄, 30.0 mol% (0.003 mol, 246.1 mg) of NaOAc (anhydrous) and 2-(5-(hydroxymethyl)furan-2-yl)-1,3-dioxan-5-ol (0.01 mol, 2.0 g). Then (0.099 mol, 10 mL) freshly distilled 2-MeTHF and (0.03 mol, 2.8 mL) degassed vinyl acetate is added. The Schlenk tube is sealed under argon and transferred to a preheated oil bath at 100 °C in which the reaction mixture is stirred for 10 h. Subsequently, the mixture is cooling down to room temperature and then 2-MeTHF and the excess of vinyl acetate are removed under reduced pressure on the rotary evaporator. The desired vinyl ether is purified by column chromatography using Et₂O/n-pentane (1:10/ v:v) as eluent on silica. After purification, the solvents are removed under reduced pressure on the rotary evaporator and the final compound is further dried at 40 °C at 10 mbar for 4 h. **Yield: 2.04 g, 81 %**

### Example 5:

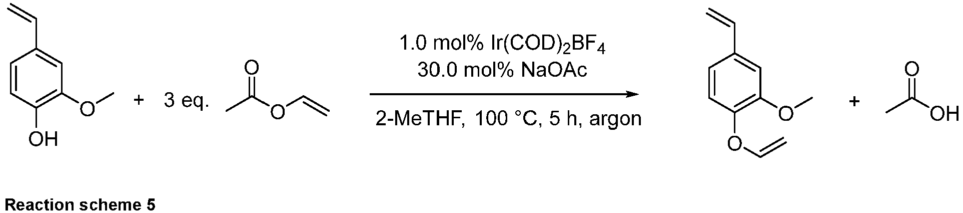

A Schlenk tube (25 mL) is charged with a magnetic stirring bar, 1.0 mol% (0.0001 mol, 50 mg) of Ir(COD)BF₄ and 30.0 mol% (0.003 mol, 246.1 mg) of NaOAc (anhydrous). Then (0.099 mol, 10 mL) freshly distilled 2-MeTHF and (0.03 mol, 2.8 mL) degassed vinyl acetate is added. Afterwards, the degassed substrate 4-vinylguaiacol (0.01 mol, 1.50 g) is added *via* syringe. The Schlenk tube is sealed under argon and transferred to a preheated oil bath at 100 °C in which the reaction mixture is stirred for 5 h. Subsequently, the mixture is cooling down to room temperature and then 2-MeTHF and the excess of vinyl acetate are removed under reduced pressure on the rotary evaporator. The desired vinyl ether is purified by column chromatography using Et₂O/n-pentane (1:10/ v:v) as eluent on silica. After purification, the solvents are removed under reduced pressure on the rotary evaporator and the final compound is further dried at 40 °C at 150 mbar for 4 h. **Yield: 1.15 g, 65%**

## Claims

1. A method for producing at least one vinyl ether compound having at least one cyclic structure, wherein the method comprises or consists of the steps:
reacting a vinyl ester with an alcohol having at least one cyclic structure and at least one hydroxy group in the presence of at least one Iridium catalyst, at least one base and at least one solvent selected from ester and/or ether solvents.

2. The method of claim 1, wherein the method relates to a method for producing at least one vinyl ether of formula (I) or (II) 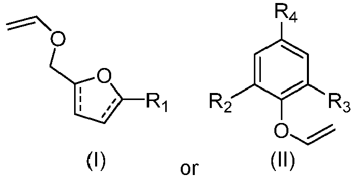 wherein
R₁ is selected from a substituted or unsubstituted C₁-C₁₀ alkyl or C₃-C₁₀ alkenyl or C₂-C₁₀ alkoxy group; "-----" can be a single or double bond; and
R₂, R₃ and R₄ are independently selected from -H, a substituted or unsubstituted C₁-C₁₀ alkyl or C₂-C₁₀ alkenyl or C₂-C₁₀ alkoxy group, with the provision that only one of R₂, R₃ and R₄ is a hydrogen;
wherein the method comprises or consists of the following steps:
reacting a compound of formula (III) 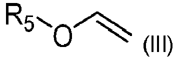 wherein R₅ is -C(=O)R₁₀, and R₁₀ is an unsubstituted C₁-C₁₀ alkyl group; preferably the compound of formula (III) is vinyl acetate,
with a compound of formula (IV) or formula (V) 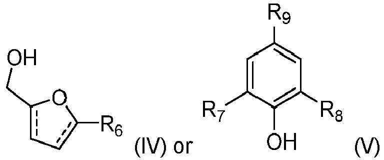 wherein
R₆ is selected from a substituted or unsubstituted C₁-C₁₀ alkyl or C₃-C₁₀ alkenyl or C₂-C₁₀ alkoxy group; "-----" can be a single or double bond; and
R₇, R₈ and R₉ are independently selected from -H, a substituted or unsubstituted C₁-C₁₀ alkyl or C₂-C₁₀ alkenyl or C₂-C₁₀ alkoxy group, with the provision that only one of R₇, R₈ and R₉ is a hydrogen;
in the presence of at least one Iridium catalyst, at least one base and at least one solvent selected from ester and/or ether solvents.

3. The method of claim 3, wherein in formula (I) and (II)
R₁ is selected from a substituted or unsubstituted C₁-C₁₀ alkyl or C₃-C₁₀ alkenyl, -CH₂-OH or - CH₂-O-CH=CH₂; -CH-(O-CH₂-)₂-CH-O-CH=CH₂; preferably R₁ is selected from -CH₃, -CH₂-OH or -CH₂-O-CH=CH₂ and/or
both "-----" are a single or a double bond,
R₂ is -H, -CH₃, or -O-CH₃ and/or
R₃ is -H, -CH₃, or -O-CH₃ and/or
R₄ is -H, -C₂H₃ or -C₃H₅;
wherein only one of R₂, R₃ and R₄ is a hydrogen; and/or
wherein in the compound of formula (IV) and (V)
R₆ is selected from a substituted or unsubstituted C₁-C₁₀ alkyl or C₃-C₁₀ alkenyl, -CH₂-OH; -CH-(O-CH₂-)₂-CH-OH; preferably R₁ is selected from -CH₃ or -CH₂-OH and/or
both "-----" are a single or a double bond,
R₇ is -H, -CH₃, or -O-CH₃ and/or
R₈ is -H, -CH₃, or -O-CH₃ and/or
R₉ is -H, -C₂H₃ or -C₃H₅;
wherein only one of R₇, R₈ and R₉ is a hydrogen;

4. The method of any one of claims 1 to 3, wherein
(i) the at least one Iridium catalyst is selected from Chlorobis(cyclooctene)iridium(I) dimer, Bis(pyridine)(1,5-cyclooctadiene)iridium(I) hexafluorophosphate, Bis(1,5-cyclooctadiene)iridium(I) tetrakis[3,5-bis(trifluoromethyl)phenyl]borate, Bis(1,5-cyclooctadiene)iridium(I) tetrafluoroborate, Bis(acetonitrile)(1,5-cyclooctadiene)iridium(I) tetrafluoroborate, Bis(1,5-cyclooctadiene)iridium(I) hexafluorophosphate, (Tricyclohexylphosphine)(1,5-cyclooctadiene)(pyridine)iridium(I)hexafluorophosphate, most preferably Chlorobis(cyclooctene)iridium(I) dimer and Bis(1,5-cyclooctadiene)iridium(I) tetrafluoroborate; and/or
(ii) the at least one base is selected from sodium acetate or potassium acetate; and/or
(iii) the least one solvent is selected from lactons, like gamma-valerolacton, ether solvents, like cyclopentyl methyl ether, methyl tert-butyl ether, tetrahydrofuran, 2-methyl tetrahydrofuran, 3-methyl tetrahydrofuran, 2,5-dimethyltetrahydrofuran, 2,2,5,5-tetramethyltetrahydrofuran, most preferably the solvent is selected from tetrahydrofuran and 2-methyl tetrahydrofuran, especially the solvent is 2-methyl tetrahydrofuran.

5. The method of any one of claims 1 to 4, wherein vinyl ester is added in molar excess over the alcohol, preferably in a molar equivalent ratio of at least 1:1 or >1:1 (vinyl ester: alcohol).

6. The method of any one of claims 1 to 5, wherein vinyl ether being the reaction product of a vinyl ester and a alcohol having two hydroxy groups, especially compound of formula (I), is a mixture of divinyl ether and mono vinyl ether compounds, preferably the mixture comprises at least 50 wt.-% divinyl ether, more preferably at least 60 wt.-%, more preferably at least 70 wt.-%, most preferably at least 80 wt.-%, based on the total weight of the mixture.

7. The method of any one of claims 1 to 6, wherein the compound of formula (III) is bio-based.

8. A vinyl ether compound of formula (I) or (II) 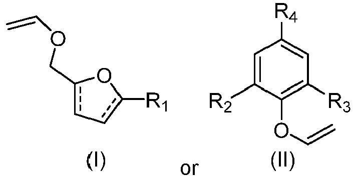 wherein
R₁ is selected from a substituted or unsubstituted C₁-C₁₀ alkyl or C₃-C₁₀ alkenyl or C₂-C₁₀ alkoxy group; "-----" can be a single or double bond; preferably R₁ is selected from a substituted or unsubstituted C₁-C₁₀ alkyl or C₃-C₁₀ alkenyl, -CH₂-OH or -CH₂-O-CH=CH₂; -CH-(O-CH₂-)₂-CH-O-CH=CH₂; preferably R₁ is selected from -CH₃, -CH₂-OH or -CH₂-O-CH=CH₂ and/or both "-----" are a single or a double bond; and
R₂, R₃ and R₄ are independently selected from -H, a substituted or unsubstituted C₁-C₁₀ alkyl or C₂-C₁₀ alkenyl or C₂-C₁₀ alkoxy group, with the provision that only one of R₂, R₃ and R₄ is a hydrogen; preferably R₂ is -H, -CH₃, or -O-CH₃, R₃ is -H, -CH₃, or -O-CH₃ and R₄ is -H, -C₂H₃ or-C₃H₅, wherein only one of R₂, R₃ and R₄ is a hydrogen

9. The compound of claim 8, wherein the vinyl ether compound is any one of the compounds of formula (VI) to (XIV)

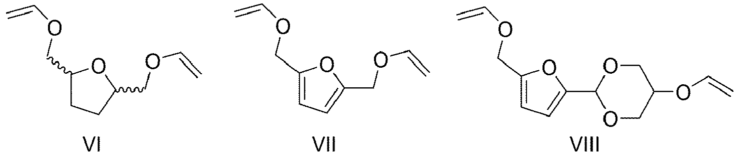

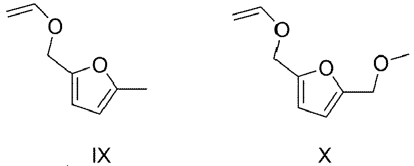

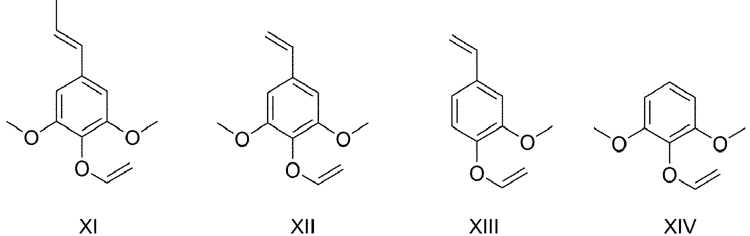

10. A polymer obtainable by polymerizing a compound of claim 8 or 9 optionally together with at least one comonomer.

11. An adhesive comprising at least one polymer according to claim 10.

12. Use of at least one compound of claim 8 or 9 or at least one polymer of claim 13 for the production of UV adhesives, cationic curings, or 1-component or 2-component systems, e.g. can coatings, epoxy- and acrylate-based coatings, structural adhesives, sealants, battery adhesives, wood coatings or pressure sensitive adhesives (PSA)).
